# EUROPEAN PATENT SPECIFICATION

(11) **EP 3 056 157 B1**
(45) Date of publication and mention of the grant of the patent: **14.03.2018**
(21) Application number: 16151522.6
(22) Date of filing: 15.01.2016
(51) Int. Cl.: A61B 17/00, A61B 17/22, A61B 17/221, A61B 17/34, A61M 25/00, A61N 1/05, A61N 1/372, A61N 1/375

(54) **A MEDICAL IMPLANT WITH A PROXIMAL RIGID FASTENER FOR INTERACTION WITH A COUPLING ELEMENT OF A CATHETER**
EIN MEDIZINISCHES IMPLANTAT MIT EINEM PROXIMALEN STARREN BEFESTIGUNGSELEMENT ZUR INTERAKTION MIT EINEM VERBINDUNGSELEMENT EINES KATHETERS
UN IMPLANT MÉDICAL AVEC UN ÉLÉMENT DE FIXATION RIGIDE, PROXIMAL POUR L'INTERACTION AVEC UN ÉLÉMENT DE CONNEXION D'UN CATHÉTER

(30) Priority: 23.01.2015 US 201562106721 P
(43) Date of publication of application: 17.08.2016
(73) Proprietor: BIOTRONIK SE & Co. KG, 12359 Berlin (DE)
(72) Inventor: Suwito, Wantjinarjo, West Linn, OR 97068 (US); Kraetschmer, Hannes, West Linn, OR 97068 (US); Muessig, Dirk, West Linn, OR 97068 (US); Whittington, R. Hollis, Portland, OR 97202 (US); Austin, Eric, Portland, OR 97221 (US); Moulder, J. Christopher, Falls Church, VA 22041 (US); von Arx, Jeffrey A., Lake Oswego, OR 97035 (US); Badie, Nima, Carmichael, CA 95608 (US); Taff, Brian M., Portland, OR 97217 (US); Dabney, Warren, Lake Oswego, OR 97035 (US)
(74) Representative: Galander, Marcus

(56) References cited:
- US-A1- 2009 082 828
- US-A1- 2013 103 049
- US-A1- 2013 116 529
- US-A1- 2013 123 875
- US-A1- 2014 257 324
- US-A1- 2014 378 991

## Description

The invention relates to a medical implant, in particular a leadless pacemaker, having a fastening arrangement for interaction with a catheter. The installation of leadless pacemakers demands surgical procedures that employ catheter-based tooling support. Such installations widely differ from the pocket-based patient interfacing associated with the implantation of traditional lead-based pacemakers. For repositioning and especially for explanting a leadless pacemaker a different tool has to be introduced into the body. In many cases it is desirable to introduce the pacemaker through the femoral and not the jugular vasculature which requires a high flexibility of the catheter.

In US 3,835,864 A a catheter is described which is used to implant an intra-cardiac stimulator through the jugularis.

WO 2012/082755 A1 describes a catheter system for retrieving a leadless cardiac pacemaker from a patient. The catheter is not suitable for implantation of the implant. The document US 2014/378991 A1 describes an implantable leadless capsule with an axial lashing rod for interaction with a catheter. The document US 2013/123875 A1 shows a leadless cardiac pacemaker with a proximal docking assembly having a T-shaped cross section. It is an object of the invention to provide a medical implant which can be delivered, implanted, repositioned as well as recaptured or explanted with the same catheter tooling. A system is provided comprising a catheter and a medical implant where delivery, implant, reposition and recapture or explant of the medical implant can be performed.

The object is achieved by the features of the independent claim. The dependent claims, the description and the drawings disclose favorable embodiments of the invention. A medical implant is proposed, for introduction into a human or animal tissue, in particular a leadless pacemaker, having a distal end and an opposing proximal end with a rigid fastener provided for interaction with a coupling element of a catheter and protruding from the proximal end in direction of a longitudinal axis of the implant, the fastener being rigidly attached to the implant. The fastener is configured for a rigid primary engagement with a coupling element of the catheter in a primary engagement mode of operation as well as to provide an interface for attaching an element provided by the catheter for a tether mode of operation and for recapturing the medical implant after the medical implant being released from catheter.

The medical implant interacts with the catheter in different modes. In a primary engagement mode, the medical implant is intimately connected to a coupling element at the catheter distal tip, for instance in a key and lock structure, and may be advanced in the blood vessel and rotated to move the implant anchor, e.g. a helix, into or out of the tissue. In a tethering mode the medical implant is released from the intimate connection to the catheter distal tip during the primary engagement mode of operation but connected via a wire loop, such as a lasso or snare, or other connection elements in order to test the location and functions of the medical implant before completely releasing it from the catheter. Further the fastener additionally provides the possibility for attaching an element for recapturing the medical implant for repositioning or explantation of the medical implant. The wire loop may be made of Nitinol, tungsten or any other biocompatible material such as a metal, polymer, or otherwise.

Favorably, the invention is suitable for helix-based or side hook medical implants, i.e. where the medical implant is anchored in the tissue by rotating the implant and/or the helix so that the helix or side hook advances into the tissue. In particular for leadless pacemaker implantation and acute explantation procedures femorally-routed catheter-based tooling support is employed. The invention solves the pivotal need for a reliable and robust interface between the medical implant and the implant/explant catheter.

The fastener of the medical implant can present a hitch geometry, so that a suite of engineered hitch geometries presented by the medical implant's distal terminus may provide adequate hand shaking with the implant/explant catheter such that torque translation in the primary engagement mode, tether modes, full decoupling, and device recapture are appropriately coordinated with various catheter articulations. Accompanying these catheter interaction needs, the hitch geometries furthermore provide topologies that mitigate risks for trauma, thrombogenesis, and entanglement in patient anatomical constructs.

The implant fastener geometries provide unique capabilities for translating torque from catheter-based implant and acute explant tools while simultaneously supporting device recapture needs. The spectrum of geometries employs minimal components, offers flexibilities for ensuring long-axis alignment between the implant and the catheter to aid in transitioning between tether-supported and primary engagements (i.e. avoiding snagging), and presents geometries to mitigate risks for either entanglement in chordae tendineae or heart wall irritation.

Due to an array of implementation complexities, leadless pacer geometric configurations, and broad-spectrum system design choices, implementing a combination tool that facilitates both implant and acute explant procedures has not been proposed nor realized within the leadless pacing field.

Favorably, a lasso-reliant implementation can be achieved which maintains an interface with the affiliated catheter, thus enabling torque translation in order to insert helix-based device anchors of the medical implant into the tissue and enabling a tether mode for decoupling the medical implant from the catheter's torque delivery mechanism in prescriptive fashion. Further, recapturing the medical implant for implant relocation and acute extraction procedures is possible with the same catheter. The fastener comprises a divot structure which allows the tether element and/or an element for recapturing, for instance the wire loop, such as a snare or lasso, when attached to the fastener, to align with the longitudinal axis of the medical implant, at least virtually, when the tether element is set under tension. The divot structure favorably avoids an undue eccentricity between catheter distal tip and medical implant because tightening of the wire loop does not cause a misalignment of the long axes of either the catheter or the medical implant. Such an undesired misalignment presents a snagging potential and can cause complications for transitioning from a tethered connection to a more robust primary engagement of the medical implant with the catheter. Primary attachments are especially valuable for designs where the device anchor demands torque translation throughout the length of the catheter to properly imbed the implant within patient myocardium.

Advantageously, the fastener incorporates a series of divots that provide a pathway for a wire loop to align into the hitch. In some cases, it may be preferred that the entire hitch be captured by the lasso, while other designs may permit the recapture of just a single lobe of the implant hitch. For cases where single lobes are captured, the geometries may blossom outward to bias the lasso loop to align as close to the center axis of the implant as possible and avoid slipping off. This alignment aids in offsetting the snagging risks as the device is recaptured and transitioned from tethered interactions toward a primary engagement. Each design additionally offers smooth geometric features to patient anatomy and lacks grossly overhanging features that would tend to permanently bind with patient valving, trabeculae, and/or chordae tendineae.

According to the invention, the fastener has generally a T-form, comprising a post attached to the proximal end and aligned with the longitudinal axis of the implant and at least one cross piece arranged fixedly at the post crosswise to the longitudinal axis, wherein the divot structure is arranged in the cross piece. In particular, the cross piece may comprise at least one lobe. Advantageously, the cross piece may be attached to the post symmetrically. In some embodiments, the cross piece may have a two-lobe configuration. The cross piece may have an oval football configuration. In a two-lobe configuration, the fastener presents larger radii of curvature to the patient anatomy than the football design. In another embodiments the cross piece may have a three-lobe or a four-lobe configuration. Favorably, the cross piece may have a rounded tip at its free ends in radial direction. Further, the cross piece may have a swollen tips to prevent the wire loop from slipping off, particularly upon cinching.

According to a favorable embodiment of the invention, the cross piece may provide a planar surface at its free end in longitudinal direction. Such a planar, table-like surface aids in transitioning from a tether mode to primary engagements with the catheter.

Rather than demanding a separate contoured feature along the housing of the medical implant to ensure lock and key mating with the catheter upon primary engagement, the configuration of each fastener and its rigidity allows for direct torque translation interfacing through the hitch. As an added bonus, in the case of the 2-lobe, 4-lobe, and certain hook hitch designs, the mating geometries within the implant/explant catheter can be identical since the plan-view footprint of the hook and 2-lobe design are subsets of a 4-lobe configuration. Such a condition means that the implant/explant tooling can be built using a 4-lobe interface, and implants can be built with either hook, 2-lobe, or 4-lobe hitches. The implant/explant tooling does not have to be redesigned. According to a favorable embodiment of the invention, the fastener may provide a twist-lock connection structure such as a bayonet-like structure for interaction with a corresponding element of the catheter. A twist-lock connection is engaged by being pushed into a socket and then twisted to lock it in place. In another additional or alternative case, the fastener may provide a fastening threaded element, like a fastening screw hole. In particular, the fastening screw hole may provide a central thread for attaching a corresponding threaded element of the catheter. The central thread may be configured as a threaded hole and the corresponding threaded element as a screw. Alternatively, the central thread may be configured as a screw and the corresponding threaded element as a threaded hole. The connection between threaded hole and screw may be utilized to tether the medical implant. A torque coil in the catheter may be used which has the property of bending flexibility and yet can still be torqued with a 1:1 ratio between proximal and distal ends of the catheter. The fastener may be configured as a mushroom-like hitch having grooves at the outside. In addition the aforementioned central threaded opening or screw for attaching a corresponding threaded element of the catheter can be incorporated in the hitch. The cross piece may be shaped as a convex spherical part attached to the post. Favorably, the grooves may be used to interact with a corresponding element such as a rotating cup at the catheter either to prevent axial rotation or to impart axial rotation. The rotating cup may be connected to a torque coil slightly larger than the torque coil used for tethering described above. One or more wire loops may protrude from the proximal end of the implant. This allows for recapturing and explantation of a medical implant in a chronic phase, i.e. several weeks after implantation when the medical implant may already be encapsulated in the patient's tissue. Favorably, the wire loops may consist of thin, flexible, radio-opaque material protruding axially and then curving radially away from the axis of the medical implant. The degree to which the flexible wire loops protrude from the medial implant greatly increases the ease of recapturing the wire loops with the catheter. The thin, flexible construction minimizes hemodynamic interference and potential damage to nearby tissue and limits the encapsulation response of the host tissue. The fastener may be a fin. The fin allows for primary engagement with a gripper tool.

The fastener may be a simple coat hanger type structure. The hook provides a notch undercut that allows for an easier recapture with the wire loop from all sides. In other words, recapture can be safely performed virtually independent from the implant and tooling relative rotational states. Further, the undercut feature of the hook supports wire loop engagement and allows for favorable axial alignment with the catheter, i.e. with minimal offset.

A catheter is proposed for handling a medical implant in a human or animal body, in particular a medical implant according to any one of the features described above, the catheter having a handle at its proximal end and a coupling element at its distal end, the coupling element being configured to provide torque to the implant for attaching the medical implant to the body tissue at a desired location in the human or animal body and for detaching, like repositioning and/or explanting, the medical implant from the human or animal body, wherein concentric sheaths are provided with an outer sheath, an inner sheath, a torque translation sheath and a cinching sheath.

In all cases, one or more from the group of the outer sheath, the inner sheath, the torque translation sheath and the cinching sheath may be configured to be steerable. In some cases the outer sheath may be a steerable outer sheath. In some cases, the inner sheath may be a steerable inner sheath. In some cases, the outer sheath as well as the inner sheath may be steerable sheaths. In other cases either the inner, outer, or both sheaths are steerable in two directions. In this case two pull wires are used, one for bending the sheath, and one for straightening it. In a further case at least one of inner or outer sheath is steerable in one direction. In this case only one pull wire is used to bend one of those sheaths with passive straightening of that sheath by reducing tension on the one pull wire. In this case the material of the steerable catheter is pre-loaded, so that it bend back in the original shape after reducing the tension. In still other cases, the inner sheath, the outer sheath, or both may be preformed to have a natural curve. In another case at least one of the sheaths is preformed to have a natural curve in one location and is steerable in a second location. In other words, a concept of a single tool is proposed that merges both implantation and acute and chronic device extraction support. It is of advantage that only few components are necessary. The larger the component count, the more opportunities a design offers to the end user for realized failure. Additionally, the grooves and contours present on known medical implant structures offer an increased risk for bacterial growth and/or thrombogenesis.

Implanting, repositioning, and explanting a medical implant such as intracardiac leadless pacemakers is challenging for several reasons. Firstly all mechanical interactions with the medical implant must occur via a catheter, secondly the mechanism used to implant the medical implant (e.g. screwing a helix into the myocardium) must be reversible for repositioning or removal, thirdly unintended tissue damage (e.g. chordae tendinae, valves' leaflets, heart walls) by the medical implant or catheter must be avoided, fourthly the natural, fibrotic capsule generated by the host tissue must be bypassed to remove a chronic implant, fifthly the implant/explant system should allow for communications with the medical implant during implant/explant with minimal interference or signal attenuation, and sixthly the implant/explant system must allow for the implant to make pacing threshold, sensing and impedance measurements before the medical implant is released. In some cases, electrical interactions with the implant may also occur via the catheter. Favorably, a lasso-reliant implementation can be achieved which maintains an interface with the affiliated catheter, thus enabling torque translation in order to insert helix-based device anchors of the medical implant into the tissue and enabling a tether mode for decoupling the medical implant from the catheter's torque delivery mechanism in prescriptive fashion. Further, recapturing the medical implant for implant relocation and acute extraction procedures is possible with the same catheter.

Having a steerable inner sheath and a separate steerable outer sheath enables the steering of the medical implant attached to the catheter in two independent planes, being particularly advantageous for femoral routing of the catheter into the ventricle. A wire element may be arranged inside the cinching sheath, the wire element providing a wire loop for capturing a fastener of the implant. The wire element has the wire loop at its free end. By moving the cinching sheath along the wire element, the wire loop can be enlarged or diminished and, when attached to the fastener of the medical implant, thus safely connected to the fastener with the cinching sheath close to the medical implant. The fastener may be locked to a key and lock structure of a coupling element of the torque translation sheath with the wire loop fixed to the fastener when the cinching sheath is retracted into the torque translation sheath. As a result, torque can be transmitted to the medical implant in this primary engagement mode and the helix of the medical implant arranged at its distal end can be turned and either removed from the tissue for releasing the medical implant from the tissue or for screwing it into the tissue for anchoring the medical implant at its location. The torque translation sheath and the cinching sheath may be configured to be completely retractable into the inner sheath. The inner sheath, the torque translation sheath and the cinching sheath may be configured to be completely or partially retractable into the outer sheath. It increases the patient's safety, if all catheter components arranged inside the inner are retracted in the inner sheath or proximally out of the inner sheath. Once the outer sheath has bent into the heart, the medical implant can then be advanced to follow the curve of the outer sheath. The torque translation sheath may be provided with a key and lock structure as a coupling element at its distal end, which key and lock structure is configured to cooperate with a fastener of the implant for transmitting torque to the implant. The key and lock structure is advantageous for re-establishing interfacing between catheter and the fastener of the medical implant in the transition from a tether mode to a primary engagement mode. In the re-enabled primary engagement mode the longitudinal axes of the catheter distal tip and the medical implant are collinear and virtually aligned. The tip of the inner sheath, which may be steerable, may advantageously provide special features that guide the medical implant into the primary engagement during transitions for tether modes. Such features can manifest tip flaring or other geometries that can collapse when the inner sheath is later pulled into the outer sheath (which also may be steerable), without influencing the outer tooling diameter. Alternatively the tip of the inner sheath may be fluted with groves to help align (either translationally or rotationally or both) the key and lock structure as the medical implant is pulled into the catheter during recapture. Preferentially the keyed interface on the catheter can freely rotate so that it will turn to align with the key on the medical device, thus avoiding unintentional dislodgement of the implant or tissue damage that may occur if the medical implant rotates. The coupling element may be configured as a cup with a twist-lock internal structure for interacting with a fastener of the implant. Alternatively or additionally the coupling element may be configured as a central thread, preferably a central screw or a central screw hole, for attaching a corresponding fastening threaded element of the implant (10). The medical implant may be tethered via the central screw and threaded hole in a tethering mode. The coupling element may be configured as a gripper. The gripper may be attached with its fingers to a finlike fastener of the medical implant. The fingers close when being pulled toward the sheath they are protruding from, gripping the fin-like fastener. A slider/rotator element may be provided at the handle for activating the coupling element, the slider/rotator element being aligned with a longitudinal axis of the catheter. The arrangement facilitates manipulation of the coupling element. A system is proposed comprising a catheter and a medical implant, where the catheter is configured to implant and recapture and/or explant the medical implant.

Favorably, a central problem to the realization of a viable leadless pacemaker design is solved as the capacity to reliably interface the implant with an affiliated catheter-based delivery and explant tooling solution is provided. As such, the distal terminus of the medical implant presents an appropriate geometry and mechanical hardware to support a robust primary connection to the implant/explant catheter; a functional tether-mode secondary connection to the implant/explant catheter; an ability to fully decouple from the implant/explant catheter and render non-irritating and non-trombogenic surfaces to patient anatomy with minimal risks for chordae tendineae entanglement, as well as device recapture in cases where repositioning or explantation are required. Advantageously the proposed fastener configurations satisfy these demands when specifically paired with a single-tool, combined, lasso-reliant implant/explant catheter. One or more sheaths may be provided for covering the medical implant during implantation and/or after recapture. A method for implantation and/or explantation of a medical implant, particular a leadless pacemaker, into a human or animal body via a catheter is proposed, comprising the steps of obtaining access to patient vasculature; routing a medical implant to an implantation location and installing the implant; assessing the medical implant operation and patient interfacing in a tether mode of the implant; releasing the medical implant from the catheter. In case a problem is detected with the medical implant, the method comprises recapturing the medical implant and either re-installing the medical implant at another location; or explanting the medical implant.

Generally, this reduces or avoids unnecessary material and production cost; inventory management and parts storage; separate tools for implant and recapture; multiple insertion, routing, and removal sequences during a single clinical procedure; added training for the operation of separate tools; and a need for developing/maintaining blood-tight valving at locations where different types of hardware enter the patient vasculature. A helix-based anchor for the medical implant can be used and critical stiffness requirements do not interfere with medical implant positioning articulations/accuracy A change between a catheter used for delivery and a catheter used for recapture or explantation is not necessary. In particular, leadless pacemaker implantation and acute explantation procedures employ catheter-based tooling support. The lengthy and somewhat torturous paths associated with the affiliated device installation and removal processes are improved by minimizing the number of instances between the commencement and completion of a given surgery where catheter-based tooling is inserted into, steered through, and removed from patient vasculature. A strategy is explored for supporting both implantation and acute extraction needs by merging such capabilities into a single tool - avoiding a need for and the risks associated with swapping between separate hardware. Further added benefits are saving on tooling development costs; eliminating a need for clinicians to interface with and learn multiple tool sets; adequately servicing use cases where leadless pacemaker implantation and acute extraction procedures occur within a common outpatient operation; and helping to reduce the risk of infection and other adverse events by minimizing the number of tools that need to be swapped in and out of the patients during the procedure. The medical implant may be covered with an inner sheath during implantation and/or after recapture and/or during explantation, thus protecting the patient and facilitating manipulating the medical implant. This inner sheath may also be steerable. The medical implant may be locked to the catheter in a primary engagement mode of operation and applying torque to the medical implant for rotating the medical implant. In particular, the medical implant may be held firmly against a key and lock structure of the catheter by tension applied to a wire element arranged in the catheter. The medical implant may be disengaged from the key and lock structure and may maintain the wire element connected to the medical implant in the tether mode of operation. A secure tether mode is possible, making the process more efficient and improving the given surgery.

According to another aspect, a method for explantation of a medical implant is proposed, particular a leadless pacemaker, having been implanted in a human or animal body, the method comprising the steps of obtaining access to patient vasculature; accessing the medical implant with a catheter, the catheter having a handle at its proximal end and a coupling element at its distal end, the coupling element being configured to provide torque to the implant for explanting the medical implant; recapturing the medical implant, and explanting the medical implant. The medical implant may be covered with an inner sheath after recapture and/or during explantation, thus protecting the patient and facilitating manipulating the medical implant. This inner sheath may also be steerable. The medical implant may be locked to the catheter in a primary engagement mode of operation and torque may be applied to the medical implant for rotating the medical implant. In particular, the medical implant may be held firmly against a key and lock structure of the catheter by tension applied to a wire element arranged in the catheter. The present invention together with the above-mentioned and other objects and advantages may best be understood from the following detailed description, wherein is shown in:
- Fig. 1: a first system comprising a catheter and a medical implant at the distal end of the catheter;
- Fig. 2: the proximal end of the catheter of Figure 1;
- Fig. 3: a detail of a medical implant illustrating a mushroom-like fastener;
- Fig. 4: the implant of Figure 3 attached to a coupling element at the catheter distal tip;
- Fig. 5: an isometric view into the coupling element of Figure 4;
- Fig. 6: a cut view of the connection between fastener and coupling element shown in Figure 4;
- Fig. 7: a wire loop coupling element for recapturing an implant;
- Fig. 8: a coupling element for recapturing an implant;
- Fig. 9: a side view of an embodiment of a medical implant displaying a fastener having a football shape arranged at the proximal end of the medical implant;
- Fig. 10: a top view of the fastener displayed in Figure 9;
- Fig. 11: a side view of a medical implant displaying a fastener having a two-lobe shape with swollen ends arranged at the proximal end of the medical implant;
- Fig. 12: a top view of the fastener displayed in Figure 11;
- Fig. 13: a side view of a medical implant displaying a fastener having a two-lobe shape arranged at the proximal end of the medical implant;
- Fig. 14: a top view of the fastener displayed in Figure 13;
- Fig. 15: a top view of a fastener having a three-lobe shape;
- Fig. 16: a top view of a fastener having a four-lobe shape;
- Fig. 17: a side view of a medical implant displaying a fastener having a hook shape arranged at the proximal end of the medical implant;
- Fig. 18: a top view of the fastener displayed in Figure 17;
- Fig. 19: a side view of a medical implant displaying a fastener having a hook shape arranged at the proximal end of the medical implant;
- Fig. 20: a top view of the fastener displayed in Figure 19;
- Fig. 21: fastener parts and a medical implant before joining the parts;
- Fig. 22: the fastener and medical implant of Figure 21 connected;
- Fig. 23: a side view of the fastener of Figure 9 with a wire loop attached;
- Fig. 24: a top view of wire loop and fastener of Figure 23;
- Fig. 25: a side view of the fastener of Figure 13 with a wire loop attached;
- Fig. 26: a top view of wire loop and fastener of Figure 25;
- Fig. 27: a side view of the fastener of Figure 19 with a wire loop attached
- Fig. 28: a top view of wire loop and fastener of Figure 27
- Fig. 29: a cut top view of the two-lobe fastener of Figure 13 in a primary engagement mode of operation with a coupling element of the catheter;
- Fig. 30: a cut top view of the four-lobe fastener of Figure 16 in a primary engagement mode of operation with a coupling element of the catheter;
- Fig. 31: a cut top view of the simple coat hanger type structure of Figure 19 in a primary engagement mode of operation with a coupling element of the catheter;
- Fig. 32: a flow chart of patient interactions with a medical implant applied with an implantation/explantation catheter;

- Fig. 33: a flow chart of patient interactions with a medical implant applied with an implantation/explantation catheter;

- Fig. 34: a longitudinal cut through a distal end of a catheter where no medical implant is attached;
- Fig. 35: a side view of a medical implant and a catheter in a primary engagement mode of operation;

- Fig. 36: a side view of a medical implant and a catheter in a tether mode of operation;

- Fig. 37: a side view of a medical implant and a catheter in a recapture mode of operation;

- Fig. 38: a side view of a recapturing operation;
- Fig. 39: a side view of the recapturing operation of Figure 38 with a wire loop attached to a medical implant;
- Fig. 40: a side view of the recapturing operation of Figure 38 with the medical implant locked to the catheter distal tip with re-enabled primary engagement mode of operation;
- Fig. 41: a side view of a medical implant and a catheter where the medical implant is retracted into a steerable inner sheath;
- Fig. 42: a side view of a medical implant and a catheter where the medical implant and the steerable inner sheath are retracted into a steerable outer sheath;

- Fig. 43: the manifold of loops attached to a medical implant;
- Fig. 44: the manifold of loops of Figure 43 attached to a medical implant with one loop elongated; and
- Fig. 45: the medical implant of Figure 44 with a fastener.

In the drawings, like elements are referred to with equal reference numerals. The drawings are merely schematic representations, not intended to portray specific parameters of the invention. Moreover, the drawings should not be considered as limiting the scope of the invention.

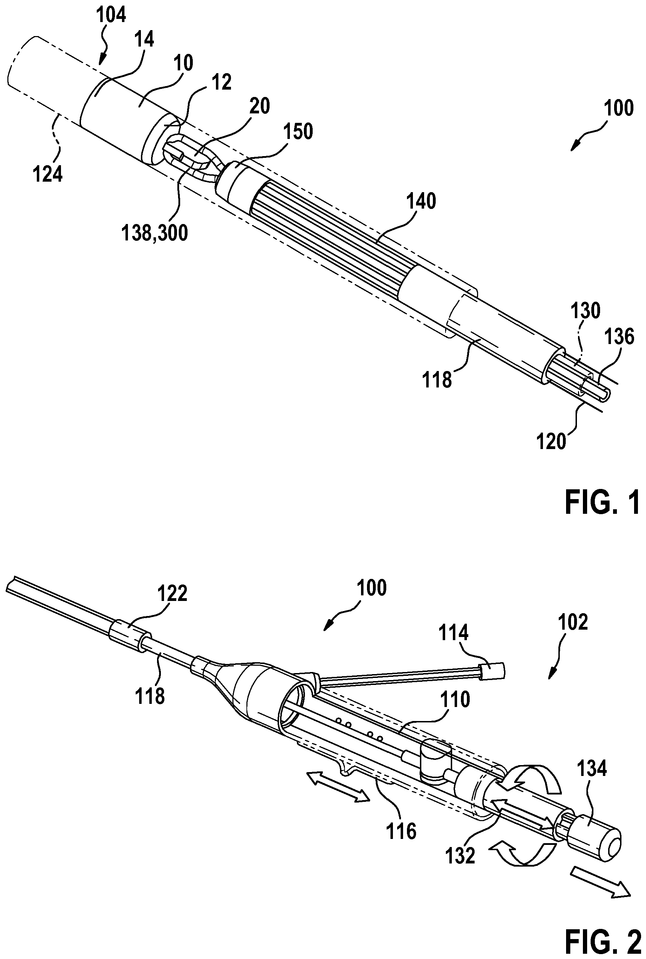

Figure 1 depicts a first system comprising a catheter 100 and a medical implant 10 at the distal end 104 of the catheter 100. Figure 2 shows the proximal end 102 of the catheter 100 of Figure 1 showing details of the handle 110. The components are partially removed in the Figure to show the inner parts of the catheter 100. The catheter 100 can be used for implantation, repositioning, as well as explantation of the medical implant. In particular, the medical implant may be a leadless pacemaker.

The medical implant 10 has a distal end 14 and a proximal end 12. The distal end 14 can be attached to the surrounding tissue (not shown) by means of an anchor such as a helix or the like (not shown) which can be screwed into the tissue (not shown) by rotating the medical implant 10. The catheter 100 is provided to advance the medical implant 10 and to rotate the medical implant 10. In order to transmit torque to the medical implant 10 the medical implant 10 is intimately attached to a coupling element 300 at the catheter distal tip via a fastener 20 arranged at the proximal end 12 of the medical implant 10. In this case, the fastener 20 is a modified flat fin, but may be a ball or a flexible neck. For the fin as fastener 20 the coupling element 300 may be a gripper 138 protruding from a metal tip 150. The gripper 138 is closed or opened by action of a gripper sheath 130.

The fin has two notches which allow for a secure attachment of the gripper without excessive compression. Each of the fingers of the gripper 138 has two appendages at its fingertip. When the fingers are pulled toward the gripper sheath 130, the gripper 138 closes while gripping the flat fin. The finger appendages prevent accidental dislodgement. A protection sheath 124 is then pushed distally with a protection sheath actuator 122, e.g. a slider, arranged at the handle 110 to cover the catheter distal tip as well as the medical implant 10 thus covering any sharp elements of the medical implant 10. The gripper torsion coil 136 and its sheath 130 are housed in a steerable sheath 140. Inside the steerable sheath 140 the large center lumen houses the gripper torsion coil 136 in its sheath 130 and two small lumen arranged diametrically outside the gripper sheath 130 house the steering wires 120 of the steerable sheath 140 which are activated by a steering actuator 116 at the handle 110. A reinforced tube is provided as an outer sheath 118 of the catheter 100.

The handle 110 depicted in Figure 2 is depicted partially transparent in order to show the inner parts of the handle 110. The protection sheath actuator 122 is arranged just outside the steerable sheath 140. The protection sheath actuator 122 allows for the catheter 100 to be bent to one side or the opposing side (left or right in the Figure) at the distal end 104. The sheath actuator 132 for the sheath 130 coupled to the gripper 138 and the element actuator 134 for activating the gripper 138 are arranged at the central lumen of the handle 110. An irrigation port 114 is arranged at the side of the handle 110. The gripper actuator 134 is attached to the gripper torsion coil 136. The element actuator 134 can slide axially in and out the gripper sheath actuator 132. Both can slide axially in and out the proximal end of the handle 110. The element actuator 134 is keyed to the sheath actuator 132 to prevent independent axially rotational movements. Rotating the gripper sheath actuator 132 will rotate the element actuator 134 as well which occurs when the medical implant 10 is to be anchored in the tissue (not shown).

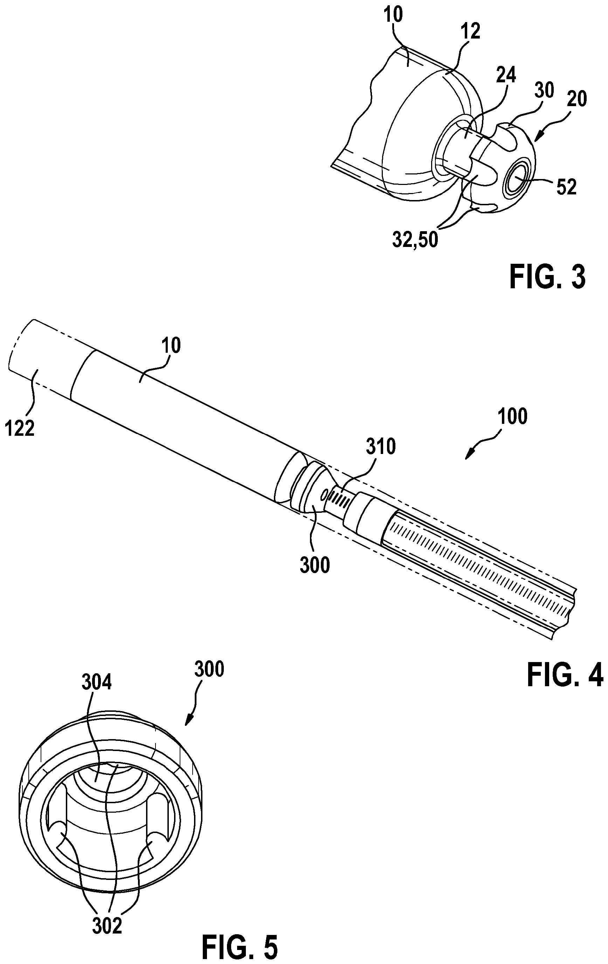

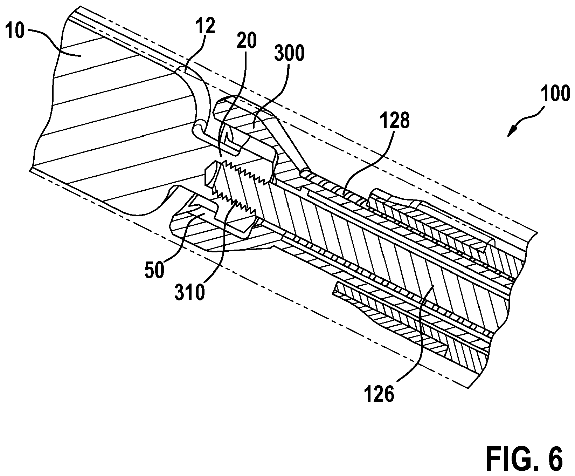

Figures 3 to 6 describe another medical implant 10. Figure 3 shows a detail of the medical implant 10 illustrating a mushroom-like fastener 20 instead of a modified fin while Figure 4 depicts the medical implant 10 of Figure 3 attached to a coupling element 300 at the catheter distal tip of a catheter 100. Figure 5 depicts an isometric view into the coupling element 300 of Figure 4, and Figure 6 shows a cut view of the connection between fastener 20 and coupling element 300 shown in Figure 4.

The mushroom-like fastener 20 consists of a post 24 attached to the proximal end 12 of the medical implant 10. A cup-like cross piece 30 with its convex side pointing away from the proximal end 12 has a multitude of divots 32 in its outer surface. The cup-like cross piece with divots 32 presents a gear structure 50, e.g. a twist-lock connection structure, for interaction with a corresponding element 300 of the catheter 100. The cup-like corresponding element 300 provides protrusions 302 protruding from its inner surface which fit in divots 32 of the fastener 20. A threaded element like a screw 310 can penetrate through a central opening 304 in the element 300 for contacting the threaded hole 52 in the fastener 20. The hole may be shallow with a depth between 1 mm to 2 mm, preferably between 1 mm and 1.5 mm.

Further, the divots 32 may be used to accommodate a wire loop in a recapture and/or tether mode when the wire loop is arranged around the post 24 and tensioned. This allows for an alignment of the longitudinal axes of the medical implant 10 and the catheter distal tip with minimal eccentricity. A protection sheath 122 may cover the medical implant 10 and the catheter 100.

The mushroom-like fastener 20 has a threaded hole 52 in its center. The catheter 100 can be connected to the fastener 20 by screwing a screw 310 into the threaded hole 52. This connection allows to tether the medical implant 10. A first torque coil 126 with the screw 310 at its distal end may be used to tether the medical implant 10, where the torque coil 126 provides sufficient bending flexibility and at the same time can still be torqued with a 1:1 ratio between its proximal and distal ends. In a favorable case, for ensuring that the medical implant 10 is not unscrewed from the tissue when releasing the medical implant 10, two torque coils are employed, one for releasing the medical implant 10, and a second one for keeping the medical implant 10 from unscrewing from tissue. These would be nested torque coils, one very small to be the tether, and that is inside a larger one which screws the medical implant 10 in and out of tissue, and keeps it from rotating when the inner torque coil is turned.

The cup-like element 300 may be also attached to one or more torque coils 128 arranged outside the first torque coil 126. These torque coils 128 may consist of two or three opposing wound wires. The catheter 100 may be similar to the catheter described in Figures 1 and 2. In such a case, the sheath actuator 132 and the element actuator 134 (Figure 2) are not keyed to each other. The sheath actuator 132 is rotated to anchor the medical implant 10 with its helix protruding from the distal end of the medical implant 10 (not shown) at its designated location, while the element actuator 134 just follows. The protection sheath 124 and the cup-like element 300 can be pulled back to see if the medical implant 10 is securely anchored. Once secured, the element 300 can be replaced, the tether screw 310 removed and the catheter 100 removed as well.

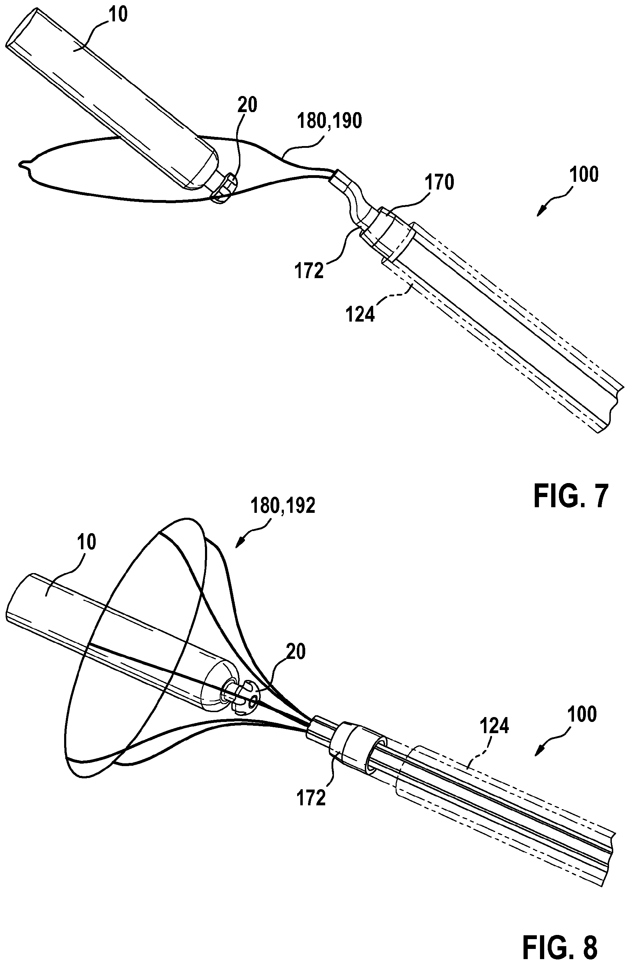

In alternative cases, Figures 7 and 8 depict retrieval tools for recapturing the medical implant 10 via its fastener 20 shown in Figure 3. Figure 7 illustrates a snare coupling element for recapturing the medical implant 10. Figure 8 illustrates a coupling element for recapturing the medical implant 10. In these cases, a separate implantation tool is needed. Figure 7 and 8 do not include a means for cinching the wire loop at the end of an extended tether.

The catheter 100 may be configured in similar way as described in Figures 1 to 2 or 3 to 6, for instance, or in any other suitable way. The center portion of the catheter 100 is replaced with wire element 180 such as a wire loop forming a wire loop 190. Pulling the wire loop around the fastener 20 and slowly bringing a distal tip 170 to the catheter 100 close to the fastener 20 would snare the medical implant 10 at the fastener 20. Pushing a wire loop sheath 172 distally would stiffen the joint, allowing for rotating the helix of the medical implant 10 to de-anchor (not shown). The protection sheath 124 is pushed distally to cover any sharp elements of the medical implant 10. Once secured, the medial implant 10 can be safely explanted.

Figure 8 depicts an alternative retrieval tool comprising a wire basket 192 as wire element 180 with a multitude of elastic wire fingers (six in the Figure) and a smaller wire, forming a 360° loop, welded at the tips. The wire finger tips are rounded, for instance of spherical shape, together with the loop to prevent accidental injury to the inner lining of the vasculature. They also provide an undercut below the cup-like cross piece (30 in Figure 3) of the fastener 20 when collapsed to pull out the medical implant 10. The collapsible wire fingers fall in the divots (32 in Figure 3) of the fastener 20 when retracted. This provides the rotational capability to de-anchor the helix of the medical implant 10.

It is possible to combine the implantation tools described in Figures 1-6 with the retrieval tools described in Figures 7 and 8, for instance by replacing the screw (310 in Figures 4, 6) with a snare wire..

The steerable catheter 100 as described above has the advantages that it may be used as implantation/explantation catheter from the femoralis, not just the jugularis. The steerable mechanisms employed are basic that can be shared with different concepts for fastener 20 and corresponding element 300 and even wire elements 180. A further advantage is that the axial rotation for anchoring or de-anchoring the helix of the medical implant 10 is in the center of the steerable catheter 100, so that it is not necessary to rotate the whole handle of the catheter 100.

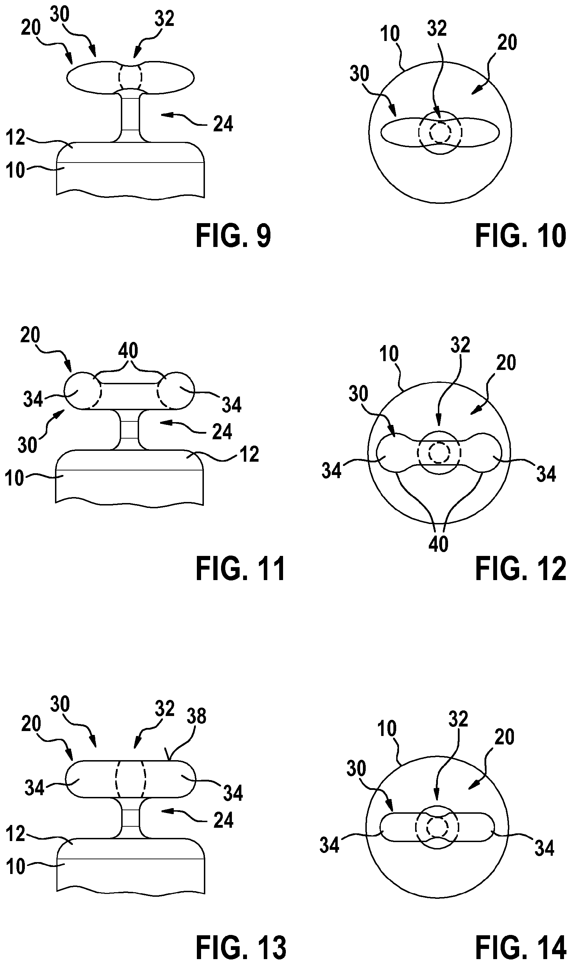

Figures 9 to 20 illustrate various medical implants 10 with fasteners 20 intended to cooperate with an implant/explant catheter having a corresponding coupling element for coupling to the fastener 20, in particular which allows for a primary engagement mode where the medical implant 10 is intimately connected to the catheter so that torque can be transmitted to the implant 10, for a functional tethering mode with a secondary connection of the medical implant 10 to the catheter, for completely decoupling from the catheter and render non-irritating and non-trombogenic surfaces to the patient anatomy with minimal risk for chordae tendineae entanglement, and for recapturing the medical implant 10 after release from the catheter when repositioning or explantation are required. The catheter may be configured similarly to the catheter 100 described in the previous Figures or as described in the following Figures or in any other suitable catheter configuration. The fasteners 20 of Figures 9 to 16 are very similar so that mainly the differences between them are highlighted. A wire loop can be arranged around a post 24. For instance, the loop can be pulled tight by pushing a cinching sheath of the catheter distally (not shown)

In particular, Figure 9 shows a side view of a medical implant 10 displaying a fastener 20 having a football shape arranged at the proximal end of the medical implant 10, while Figure 10 shows a top view of the fastener 20 displayed in Figure 9. The fastener 20 provides a full hitch capture by a wire loop provided by a catheter. The fastener 20 is composed of a rigid support post 24 and a cross piece 30 at the free end of the post 24 and has generally a T-shape. A divot 32 is arranged in the center of the cross piece 30 in direction of the longitudinal axis of the post 24. The cross piece 30 in this embodiment has the shape of an American football. When a wire loop is attached, the tips of the cross piece 30 prevent the wire loop from slipping from the cross piece 30.

Figure 11 shows a side view of a medical implant 10 displaying a fastener 20 having a two-lobe shape with swollen ends arranged at the proximal end of the medical implant 10, while Figure 12 shows a top view of the fastener 20 displayed in Figure 11. The cross piece 30 exhibits a two-lobe configuration. The tips 40 of the cross piece 30 are swollen to prevent a wire loop from slipping off upon cinching the cinching sheath (not shown).

Figure 13 shows a side view of a medical implant 10 displaying a fastener 20 having a two-lobe shape arranged at the proximal end of the medical implant 10, while Figure 14 shows a top view of the fastener 20 displayed in Figure 13. The cross piece 30 exhibits a two-lobe configuration. The tips are fatter than the divot 32. The cross piece 30 shows a planar surface 38 in longitudinal direction.

The two-lobe configurations present larger radii of curvature to the patient anatomy than the football design. Via the blossomed tips 40 of the lobe features on the two-lobe configuration, both single lobe and hole hitch recapture are facilitated because cinching of the implant/explant wire loop does not cause the fastener 20 to slip out of the tooling.

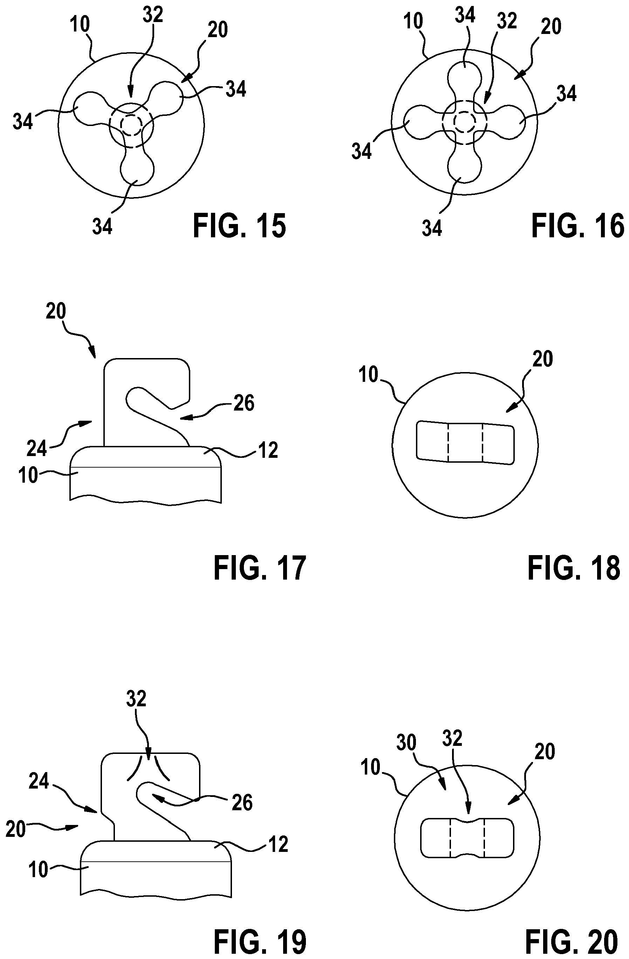

Figure 15 shows a top view of a fastener 20 having a three-lobe shape. Figure 16 depicts a top view of a fastener 20 having a four-lobe shape. Each of the designs provide an upper table-like surface to aid in transitioning from tether modes to primary engagement with the catheter.

Figure 17 illustrates a side view of a medical implant 10 displaying a fastener 20 having a hook shape arranged at the proximal end of the medical implant 10, while Figure 18 shows a top view of the fastener 20 displayed in Figure 17. The V-shaped, protected undercut of the hook ensures alignment with the longitudinal axis of the implant/explant catheter to minimize concerns for unintended binding when transitioning implants 10 form tether- to primary-mode engagements.

Figure 19 shows a side view of a medical implant 10 displaying a fastener 20 having a hook shape arranged at the proximal end of the medical implant 10 while Figure 20 shows a top view of the fastener 20 displayed in Figure 19. Generally, the hook configuration can be considered as a fin configuration (e.g. Figure 1) with an undercut.

The V-shaped, protected undercut of the hook ensures alignment with the longitudinal axis of the implant/explant catheter to minimize concerns for unintended binding when transitioning implants 10 form tether- to primary-mode engagements. The undercut 26 at the post 24 provides an easier re-engagement with the implant/explant recapture wire loop from all sides of the fastener 20. As a result, recapture is virtually independent from the relative rotational orientation of the medical implant 10 and the catheter.

The top down footprint of the hook configuration is similar to the two-lobe configuration described above which enables primary engagement with the catheter with the same design of the corresponding coupling element on the catheter side.

The fasteners 20 described provide large translational and/or rotational degrees of freedom when paired with the implant/explant catheter using less complexity and only few discrete components. The mechanical rigidity and lobe-dependent or undercut-dependent topologies offer a means for translating torque which supports the inclusion of helix-based myocardial interfacing strategies of medical implants 10. The reduced complexity and component count additionally offer fewer opportunities for implant hitch ' failure, improving safety/reliability without compromising core functional capabilities.

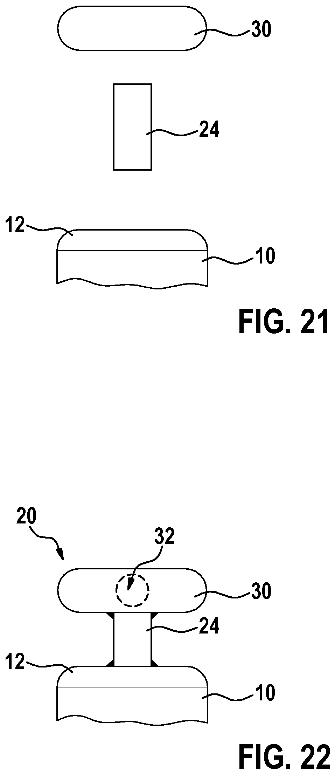

Figures 21 and 22 illustrate a conceivable assembly process for pairing a medical implant 10 and a fastener 20. For the sake of simplicity, only a two-lobe configuration is shown. The fastener's components, i.e. a post 24 and a cross piece 30 may be manufactured as separate components and then weld attached so that a weld seam is arranged between the post 24 and the cross piece 30 and the post 24 and the medical implant 10.

Post 24 and cross piece 30 may be manufactured of cut wire and the tips of the cross piece 30 rounded. The divot 32 may be cut either before or after assembly.

In an alternative case, post 24 and cross piece 30 may be manufactured as a single assembly and then weld attached to the medical implant 10.

In a further alternative case, implant 10 and fastener 20 may be manufactured as a single assembly, built as a single, monolithic piece of hardware. No weld seams are necessary at all.

In a further alternative case, post 24 may be integrated in the medical implant 10 and the cross piece 30 weld attached to the post 24.

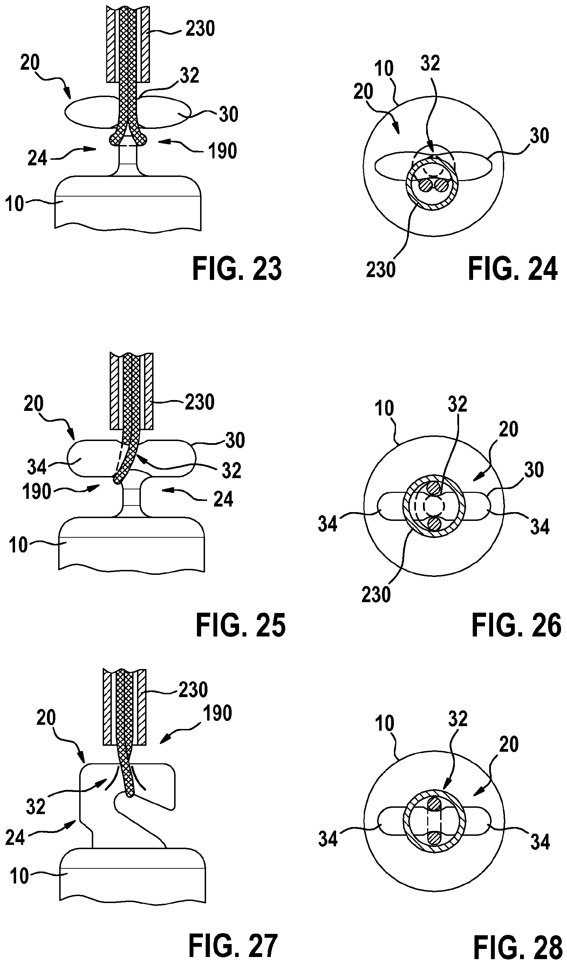

Figures 23 to 28 depict distinct methods for capturing a medical implant 10 via its fastener 20 with a wire loop 190. In particular, Figure 23 shows a side view of the fastener 20 of Figure 9 with a wire loop 190 attached and a cinching sheath 230 pushed distally towards the fastener 20, providing a full capture of the entire fastener 20 (hitch 20). The wire loop 190 is wrapped around the full post 24. Figure 24 shows a top view of wire loop 190 which aids to reduce an eccentricity and fastener 20 of Figure 23. The divot 32 and the fat ends of the cross piece 30 prevent the wire loop 190 from slipping off the cross piece 30. Figures 25 and 26 illustrate capturing by one lobe of the fastener 20. Figure 25 shows a side view of the fastener 20 of Figure 13 with a wire loop 190 attached to one lobe and Figure 26 shows a top view of wire loop 190 and fastener 20 of Figure 25. The divot 32 and the fat ends of the cross piece 30 prevent the wire loop 190 from slipping off the cross piece 30. The wire loop 190 is wrapped around only one of the lobes of the fastener 20. Due to this and the divot 32 the eccentricity between the medical implant and the catheter axis is very small so that the components are virtually aligned to each other, thus aiding in transitions from tether modes to primary engagement. The fat ends prevent the wire loop 190 from slipping off the cross piece 30.

It should be mentioned that, although only a two-lobe configuration is illustrated, the features described therein are applicable to a three-lobe and four-lobe configuration as well.

Figures 27 and 28 depict engagement with a hook configuration of fastener 20. Figure 27 depicts a side view of the fastener 20 of Figure 19 with a wire loop 190 attached and Figure 28 shows a top view of wire loop wire loop 190 and fastener 20 of Figure 27. The wire loop 190 is wrapped around a point that is aligned with the longitudinal center axis of the medical implant 10. There is no eccentricity between the catheter and the medical implant 10.

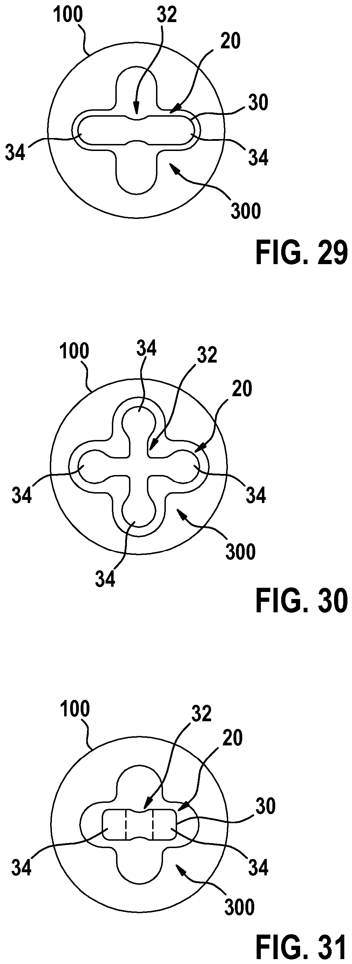

Figures 29 to 31 illustrate a coupling element 300 of an implant/explant catheter 100 corresponding to a fastener 20 of a medical implant 10, wherein the fastener 20 comprises a cross piece 30. An advantage of using a coupling element 300 with four lobes instead of two is that it is easier to rotationally align the coupling element 300 with the medical implant 10. One important security aspect is that any unintended movement of the implant 10 has to be prevented. For example an unintended rotational movement of implant 10 may cause its dislodgement (e.g. unintended unscrewing) and/or damages of tissue (e.g. perforation of the heart wall).

The essence is that only coupling element 300 performs movements, both longitudinally through manipulating the catheter 100 in longitudinal direction and rotationally by rotating the coupling element 300 through the surgeon. Further, with the lock and key fitting design disclosed, alternatively or additionally to said active rotation a passive rotation is possible and intended to prevent from the above mentioned problems. This passive rotation established by smoothly slipping the cross piece 30 of fastener 20 into the lobes of coupling element 300, which occurs, when the catheter 100 is pushed forward in proximal direction and the cross piece 30 of the implant's fastener 20 overlap with the protrusions 302 of the coupling element 300. The smooth surface and shape of the cross pieces 30 cause a smooth rotation of coupling element 300 and during pushing the catheter in proximal direction said cross piece 30 slips into the lobes between the protrusions 302. The more lobes the coupling element 300 has, the easier the engagement of implant's cross piece 30 occurs and the rotational movement is less. If the coupling element 300 has two lobes, then the coupling element 300 might have to rotate by up to 90 degrees to rotationally align with the coupling element 300. With four lobes the coupling element 300 only has to rotate up to 45 degrees. If the coupling element 300 has eight lobes, then only up to 22.5 degrees of rotation will be necessary to align the lock and key fitting. In general the more lobes in the coupling element 300 the better from a rotation point of view.

Figure 29 shows a cut top view of the two-lobe fastener 20 of Figure 13 in a primary engagement mode of operation with a coupling element 300 of the catheter. Figure 30 shows a cut top view of the four-lobe fastener 20 of Figure 16 in a primary engagement mode of operation with a coupling element 300 of the catheter, while Figure 31 presents a cut top view of the hook fastener 20 of Figure 19 in a primary engagement mode of operation with a coupling element 300 of the catheter. Favorably, the two-lobe configuration, the four lobe configuration and the hook configuration all fit with the same coupling element 300, so that the same lock and key feature in the catheter is compatible with said configurations which allows primary engagement of the medical implant 10 with the implant/explant catheter.

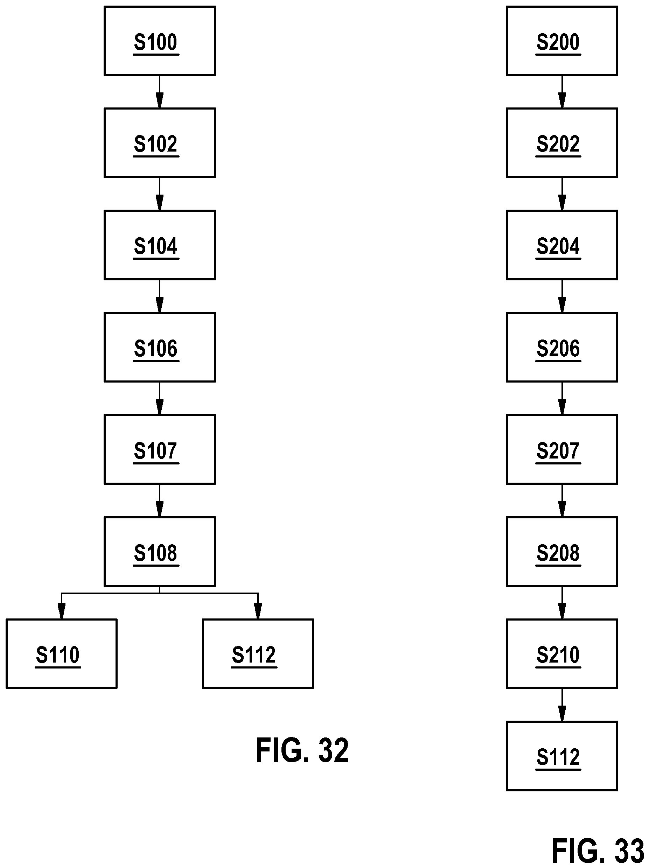

Figure 32 illustrates a flow chart of patient interactions with a medical implant applied with an implantation/explantation catheter. In step S100 access is obtained to a patient vasculature for a medical implant 10, in particular a leadless pacemaker, which is advanced via an implant/explant catheter. In step S102 the medical implant is routed to the heart and installed. In step S104 the operation of the medical implant and the patient interfacing are assessed in a tether mode of operation. In step S106 the medical implant is released from the implant/explant catheter. Subsequently, in step 107 it is assessed whether a problem exists with the medical implant. If subsequently something problematic is noticed in a monitoring test such as a fluoro imaging, the medical implant is recaptured in step S108 with the implant/explant catheter. Depending on the assessment of the detected problem, the medical implant may either be re-installed elsewhere in step S110 or may be explanted in step S112.

Favorably, there are only few steps necessary for removing or reinstalling the medical implant. In particular, the medical implant is a helix-based or side hook based implant to anchor.

Figure 33 illustrates a flow chart of patient interactions with a medical implant applied with an explantation catheter, where the patient vasculature is accessed months after an implantation has occurred and subsequently the medical implant is removed.

In step S200 access is obtained to a patient vasculature where a medical implant, in particular a leadless pacemaker, had been implanted previously. In step S202 the catheter is routed to the medical implant. In step S204 an un-cinched wire loop (or another retrieval tool described above) is placed over a fastener of the medical implant and a tether mode of engagement is subsequently established by cinching the wire loop in step S206, thus securing the capture of the medical implant at the catheter's distal end. By either advancing the catheter and/or tensioning the wire loop and its cinching feature in coordinated fashion, the system transitions from a tethered mode of implant interfacing to one where a lock and key primary engagement is re-established in step S208. In S210, the tooling can translate torque to the medical implant to explant it. The tip of the torque transmitting sheath may provide features that guide the fastener of the medical implant into the primary engagement with the coupling element in step S212 and allow removing the medical implant.

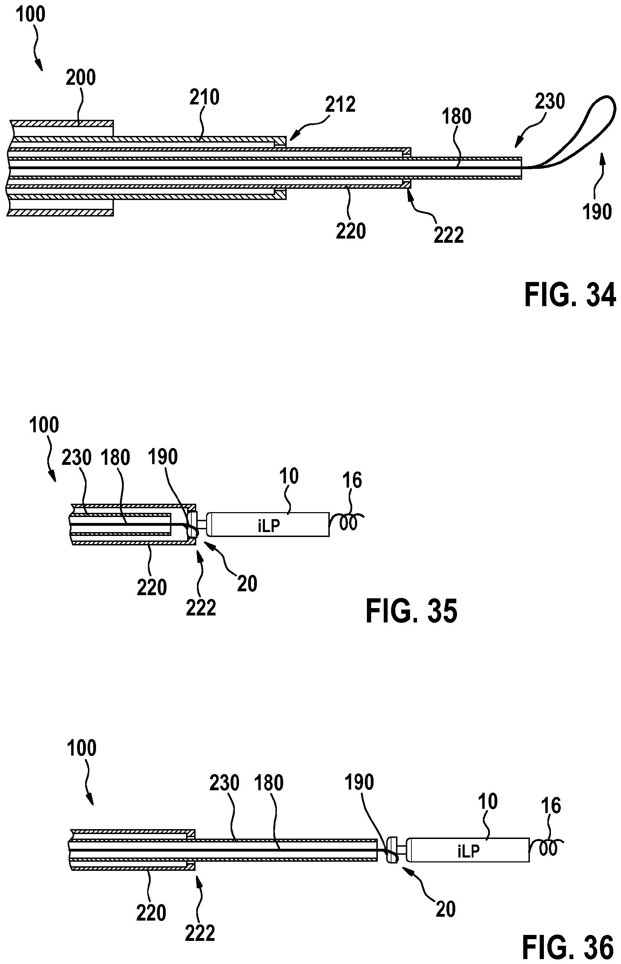

Figures 34 to 42 illustrate in a simplified way modes of operation of an implantation/explantation catheter 100 interacting with a medical implant 10. These figures additionally highlight the critical mechanisms and design elements necessary for the implementation of an all-in-one catheter tool.

Figure 34 illustrates a longitudinal cut through a distal end of a catheter 100 without a medical implant attached. The catheter enables implantation as well as acute explantation of medical implants, in particular leadless pacemakers, which rely upon helix-based patient interfacing anchors. One catheter-based tool is a wire 180 with a wire loop 190 at its end.

In one case the catheter 100 comprises a steerable outer sheath 200, surrounding a steerable inner sheath 210 that surrounds a torque transmitting sheath 220 which encloses a cinching sheath 230. Inside the cinching sheath 230 a wire element 180 is arranged with a wire loop 190 at its free end. The outer sheath 200 and the inner sheath 210 may be equipped with soft tips. The torque transmitting sheath 220 has lock and key features 222 as coupling element at its distal end which are intended to accommodate the fastener of the medical implant and to deliver torque thereto. Having a steerable outer sheath 200 and a separate steerable inner sheath 210 enables steering of the device in two planes, and it allows the two steering points to be moved with respect to each other, which is particularly favorable for femoral routing of the catheter 100.

It is to be understood, however, that in some cases the outer sheath 200 and cinching sheath 230 may be eliminated and implantation and acute explantation can thus be supported with still less complex hardware.

As shown in a simplified way in Figure 35, to torque helix-dependent medical implants 10 into the desired location, for instance for torqueing the helix of a leadless pacemaker into the heart, the wire element 180 within the catheter 100 serves to retain the fastener 20 of the medical implant 10 within or in close proximity to mating lock and key structures 222 (or a series of such mating interfaces) at the distal tip of the implant/explant catheter 100. This keyed interfacing as well as special structures internal to the catheter 100 ensure that rotations administered by the clinician at the catheter's proximal end translate into device rotations at the interface between the implant anchor and the heart wall. The depicted arrangement of catheter 100 and medical implant 10 represents a primary engagement mode of operation.

In a next step of the implantation procedure tethered modes of operation are used, which is shown in Figure 36 in a simplified way. While torque transmitting sheath 220 is retracted internal wire loop 190, as well as an affiliated cinching feature such as a cinching sheath 230 remain engaged with the fastener 20 of the medical implant 10. In this situation the keyed interface between fastener 20 of the medical implant 10 and mating lock and key structures 222 is disengaged and a torqueing movement of the implant 10 is not possible. While the internal wire loop 190 is still engaged with fastener 20 of implant 10, the primary engagement mode of operation as shown in Figure 35 can easily be re-established.

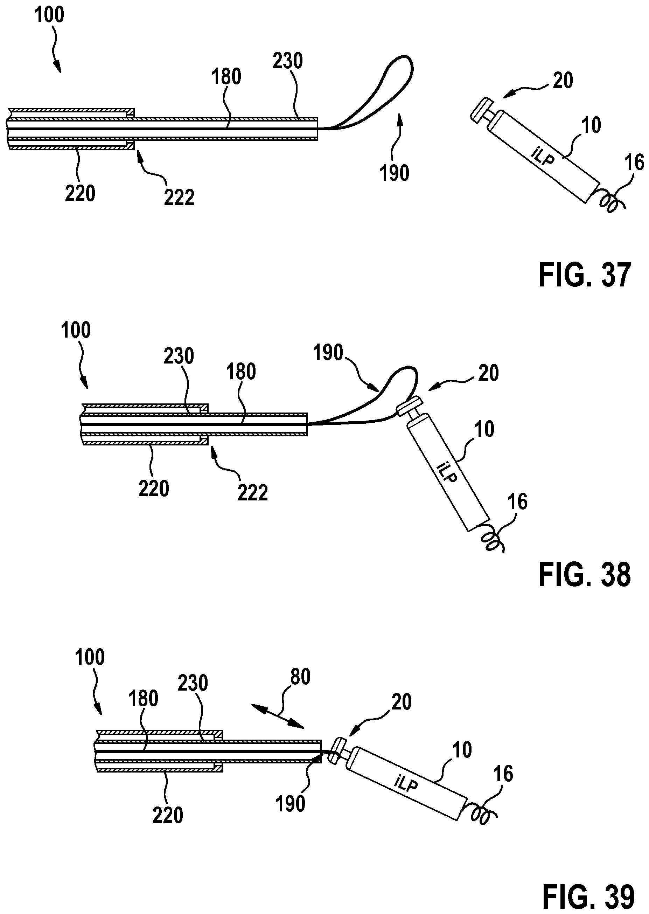

By retracting the wire loop cinching feature alone, the wire loop opens to enable a complete decoupling of the medical implant 10 from the catheter-based tooling (wire loop 190), as shown in Fig. 37.

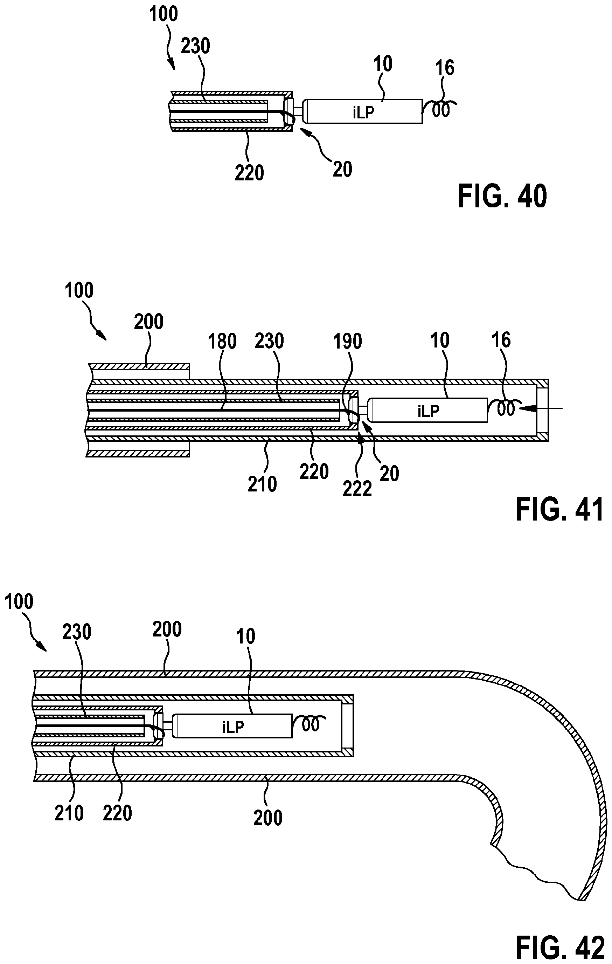

For recapture, indicated in Figures 38 to 40, the un-cinched wire loop 190 is placed over the fastener 20 of the medical implant 10 as shown in Fig. 38. A tether mode of engagement is subsequently reestablished by cinching the wire loop 190, as shown in Figure 39. This cinching forces the wire loop 190 to lock with fastener-associated divots/features like the cross piece or hook 30 (as shown in Figures 3, 9 to 20, 23 to 31) that, in turn, ensure securing capture of the medical implant 10 at catheter's distal end. By advancing the catheter 100 in proximal direction, the system readily transitions from a tethered mode of implant interfacing to one where the lock and key primary engagement mode is re-established. This is shown in Figure 40. From this condition, the tooling can again translate torque to the medical implant 10 to either reposition it or to explant it.

The tip of the torque transmitting sheath 220 may provide features that guide the fastener 20 of the medical implant 10 into the primary engagement with the coupling element 300. This guide helps to align the implant 10 to the catheter 100 (explant tool) both longitudinally and rotationally. To help align the implant 10 to the catheter 100 (explant tool) longitudinally, in one case the lock and key mechanism is fluted, to help allow engagement of the fastener 20 at a broad range of angles, and then force alignment as the two are pulled together. This full suite of articulations has been incorporated into a single tool providing a consolidated design strategy.

Figure 41 and Figure 42 show inner sheathing protecting and shielding the anchor (helix 16) of the medical implant 10. The medical implant 10 can be completely retracted into the inner sheath 210 so that it is protected from patient interfacing by the inner sheath 210 (Figure 41). In one case the medical implant 10 and the inner sheath 210 can retract back internal to the outer sheath 200 for some centimeters, e.g. 10 centimeters (Figure 42). This configuration allows the outer sheath 200 to make tighter turning radii to facilitate ease of entry into the heart from the coronary vessels. Once the steerable outer sheath 200 has bent into the heart, the medical implant 10 can then be advanced to follow the curve of the outer sheath 200. The disadvantage of allowing the inner sheath to fully retract into the outer sheath is that it requires the outer sheath to be a larger diameter so that it can accommodate the implant and the inner sheath. Alternatively, the inner sheath flares out to accommodate the implant and the outer sheath can thus be the same diameter (or lower diameter) as the flared portion of the inner sheath. This results in an implant system of thinner overall diameter, but the cost is that the inner sheath cannot be fully retracted into the outer sheath.

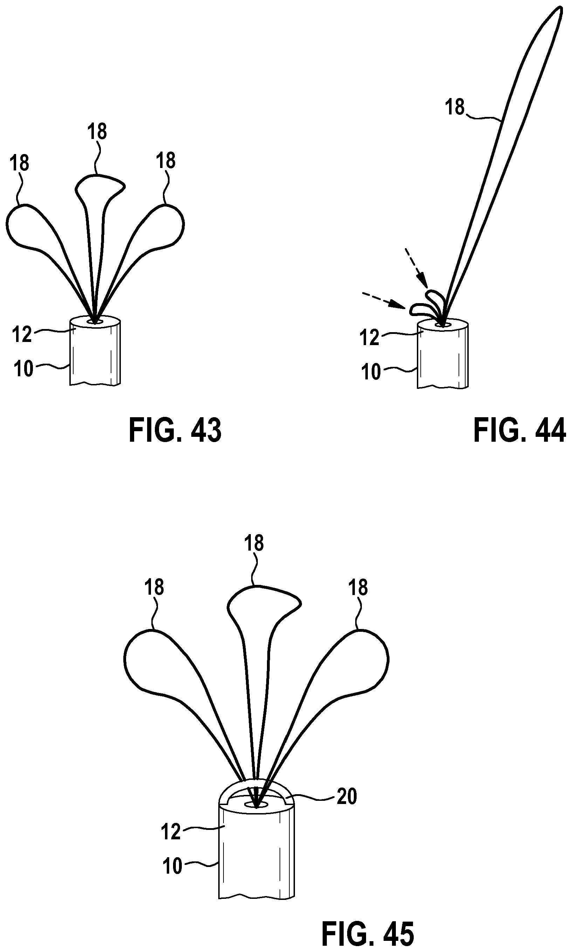

The present system merges a suite of articulations and implant/explant support needs into a common framework, demonstrating a single-tool case. By leveraging a wire-loop-based strategy to retain the medical implant 10 within a keyed interface inside the catheter 100, the system supports primary engagement modes. That same wire loop 190, when paired with a cinching feature 230, additionally enables both tether modes as well as recapture of the medical implant 10, thus merging capabilities that are either assigned to a separate tool or left unsupported in other designs. Figures 43 to 45 illustrate in schematic views another medical implant 10 which facilitates recapturing of the medical implant 10. Implanting, repositioning, and explanting intracardiac leadless pacemakers all require establishing a robust device-catheter interface. This interface in this case enables the medical implant 10 to be secured to the myocardium, permit electrophysiological characterization, and enable safe detachment of the medical implant 10 from the myocardium in both acute and chronic implant scenarios. Damage to the surrounding tissue from the interface fastener or entanglement of the fastener should be prevented due to the negative consequences to the heart. Particularly after in-growth, i.e. typically beyond 4 to 6 weeks after implantation, establishing this implant-catheter contact is complicated by the natural fibrotic encapsulation of the medical implant 10, which commonly envelopes the medical implant proximal end (i.e., terminal of the medical implant 10, distal to the myocardium).

Although the example presented in the Figures assumes a cylindrical medical implant 10 with a screw-in helix fixation mechanism, the implant-catheter interface can be applied to other configurations as well.

At the proximal end 12 of the medical implant 10, the interface includes one or more fasteners 20 (e.g. handles, loops) or fasteners as described in the previous Figures. On the catheter side, a hook will extend from the catheter to link into the arc at the proximal end of the medical implant 10 (not shown).

Several thin, flexible, radio-opaque, rabbit-ear loops 18 or snares sprout from the center of the medical implant 10, protruding axially toward the endocardium, and then curving radially away from the axis of the medical implant 10. The degree to which the flexible loops 18 protrude from the proximal end 12 greatly increases the ease of capturing them with the catheter. Although the loops 18 may be relatively large, their thin, flexible construction will minimize hemodynamic interference and potential damage to nearby tissue. Their flexibility will also limit the encapsulation response of the host tissue. The set of loop leaflets will be formed by collapsing one continuous circular wire at several points into a shared housing on the proximal end 12 of the medical implant 10, such that pulling on one loop shortens the rest, similar to a snare (Figure 43). Once one loop 18 has been captured by the catheter hook and maximally extended, the catheter's sheath can then extend to envelope the proximal end 12 of the medical implant 12 without interference from the other loops 18. Of course, in some cases a design of the loop 18 may be employed that does not collapse in this manner, in particular for delicate cases where collapse might risk entangling the patient anatomy.

A curved, rigid fastener 20 such as a handle extends from the implant proximal end 12 (Figure 44). The fastener 20 can be used for applying torque to engage/disengage the anchor of the medical implant 10 at the myocardium (e.g. using the handle as the flat-head tip of a screwdriver. On the catheter side, a sheathed, blunt hook will extend into the flexible loops 18 to establish primary contact. This primary contact can be used to draw the catheter to the medical implant 10 in preparation for the rigid fastener's secondary contact. With this closer, secondary contact, the catheter can mate with the medical implant 10 and the rigid fastener 20 can be used to apply torque (e.g. screw/unscrew the helix) while maintaining a hold on the medical implant 10. Continuously maintaining the primary contact will limit the potential for a medical implant 10 to be freely floating inside the cardiovascular system.

This interface advantageously addresses concerns associated with the implantation, repositioning, and extraction of grown-in intracardiac leadless pacemakers. Implementing a hook mechanism on the catheter, rather than on the medical implant 10 itself, minimizes the chance for the leadless pacemaker tail to become entangled with the internal structures of the heart. The smooth, curved geometry of the arc(s) on the proximal end 12 of the medical implant 10 also minimizes chronic damage and scarring of the valves and heart wall.

### List of References

- 10: implant
- 12: proximal end
- 14: distal end
- 16: helix
- 18: loop
- 20: fastener
- 22: connection
- 24: post
- 26: undercut
- 30: cross piece
- 32: divot structure
- 34: lobe
- 40: tip
- 50: bajonet structure
- 52: threaded hole
- 80: longitudinal axis
- 100: catheter
- 102: proximal end
- 104: distal end
- 110: handle
- 112: septum
- 114: irrigation port
- 116: steering actuator
- 118: reinforced sheath
- 120: steering wire
- 122: protection sheath actuator
- 124: protection sheath
- 126: torque coil
- 128: torque coil
- 130: gripper sheath
- 132: sheath actuator for gripper sheath
- 134: element actuator for gripper
- 136: gripper torsion coil
- 138: gripper
- 140: flexible sheath
- 150: metal tip
- 170: distal tip
- 172: wire sheath
- 180: wire element
- 190: wire loop, tether element, recapturing element
- 192: wire basket
- 200: steerable outer sheath
- 210: steerable inner sheath
- 212: marker
- 220: torque translation sheath
- 222: key and lock structure
- 230: cinching sheath
- 300: coupling element for fastener
- 302: protrusion
- 310: threaded element (screw), tether element

## Claims

1. A medical implant (10) for introducing into a human or animal tissue, in particular a leadless pacemaker, having a distal end (14) and an opposing proximal end (12) with a rigid fastener (20) provided for interaction with a coupling element (300) of a catheter (100) and protruding from the proximal end (12) in direction of a longitudinal axis of the implant (10), the fastener (20) being rigidly connected to the implant (10),
**characterized in that**
the fastener (20) has generally a T-form, comprising a post (24) attached to the proximal end (12) and aligned with the longitudinal axis of the implant (10) and at least one cross piece (30) arranged fixedly at the post (24) crosswise to the longitudinal axis, wherein an indentation structure (32) is arranged in the cross piece (30).

2. The medical implant according to claim 1, wherein the indentation (32) is arranged in the center of the cross piece (30) in direction of the longitudinal axis of the post (24) and wherein the indentation allows the tether element (190) and/or an element for recapturing (190, 192) when attached to the fastener (20) to align with the longitudinal axis of the medical implant (10).

3. The medical implant according to claim 1 or 2, wherein the cross piece (30 is attached to the post (24) symmetrically.

4. The medical implant according to claim 3, wherein the cross piece (30) has a two-lobe configuration (34).

5. The medical implant according to one of the claims 1 to 3, wherein the cross piece (30) has an oval football configuration.

6. The medical implant according to any one of the preceding claims, wherein the cross piece (30) has a rounded tip at each of its free ends in radial direction.

7. The medical implant according to claim 6, wherein the cross piece (30) has swollen tips.

8. The medical implant according to any one of the preceding claims, wherein the cross piece (30) provides a planar surface at its free end (38) in longitudinal direction.

9. The medical implant according to claim 8, wherein the fastener (20), preferably the free end (38), provides a fastening threaded element, preferably a central thread (52) for attaching a corresponding threaded element of the catheter (100).

10. The medical implant according to any one of the preceding claims, wherein the fastener (20) is configured for a rigid primary engagement with a coupling element (300) of the catheter (100) in a primary engagement mode of operation as well as for providing an interface for attaching a tether element (190, 310) provided by the catheter (100) for a tether mode of operation and for recapturing the medical implant (10) when the medical implant is released from catheter (100).

11. The medical implant according to any one of the preceding claims, wherein the fastener (20) provides a twist-lock connection structure (50) for interaction with a corresponding element of the catheter (100).

## Patentansprüche

1. Medizinisches Implantat (10) zur Einführung in ein menschliches oder tierisches Gewebe, insbesondere leitungsloser Herzschrittmacher, das ein distales Ende (14) und ein entgegengesetztes proximales Ende (12) mit einer steifen Befestigungsvorrichtung (20) aufweist, die für das Zusammenwirken mit einem Kupplungselement (300) eines Katheters (100) bereitgestellt ist und aus dem proximalen Ende (12) in der Richtung einer Längsachse des Implantats (10) hervorsteht, wobei die Befestigungsvorrichtung (20) starr mit dem Implantat (10) verbunden ist,
**dadurch gekennzeichnet, dass**
die Befestigungsvorrichtung (20) im Allgemeinen eine T-Form aufweist, die einen Pfosten (24), der an das proximale Ende (12) angebracht und auf die Längsachse des Implantats (10) ausgerichtet ist, und mindestens ein Querstück (30), das fixiert an dem Pfosten (24) quer zu der Längsachse angeordnet sind, umfasst, wobei eine Vertiefungsstruktur (32) im Querstück (30) angeordnet ist.

2. Medizinisches Implantat nach Anspruch 1, wobei die Vertiefung (32) im Mittelpunkt des Querstücks (30) in der Richtung der Längsachse des Pfostens (24) angeordnet ist und vorbei die Vertiefung gestattet, dass das Halteelement (190) und/oder ein Element zum Wiederaufgreifen (190,192), ist es an der Befestigungsvorrichtung (20) angebracht, sich auf die Längsachse des medizinischen Implantats (10) ausrichtet.

3. Medizinisches Implantat nach Anspruch 1 oder 2, wobei das Querstück (30) symmetrisch an den Pfosten (24) angebracht ist.

4. Medizinisches Implantat nach Anspruch 3, wobei das Querstück (30) eine Doppellappenkonfiguration (34) aufweist.

5. Medizinisches Implantat nach einem der Ansprüche 1 bis 3, wobei das Querstück (30) eine ovale Footballkonfiguration aufweist.

6. Medizinisches Implantat nach irgendeinem der vorhergehenden Ansprüche, wobei das Querstück (30) eine abgerundete Spitze an jedem seiner freien Enden in radialer Richtung aufweist.

7. Medizinisches Implantat nach Anspruch 6, wobei das Querstück (30) geschwollene Spitzen aufweist.

8. Medizinisches Implantat nach irgendeinem der vorhergehenden Ansprüche, wobei das Querstück (30) an seinem freien Ende (38) eine planare Oberfläche in Längsrichtung bereitstellt.

9. Medizinisches Implantat nach Anspruch 8, wobei die Befestigungsvorrichtung (20), bevorzugt das freie Ende (38), ein mit einem Gewinde, bevorzugt einem Zentriergewinde (52), versehenes Befestigungselement zum Anbringen eines entsprechenden, mit einem Gewinde versehenen Elements des Katheters (100) bereitstellt.

10. Medizinisches Implantat nach irgendeinem der vorhergehenden Ansprüche, wobei die Befestigungsvorrichtung (20) für ein starres primäres Einrasten in ein Kupplungselement (300) des Katheters (100) in einem primären Einrastarbeitsmodus sowie zum Bereitstellen einer Übergangsstelle zum Anbringen eines Halteelements (190, 310) konfiguriert ist, das durch den Katheter (100) für einen Haltearbeitsmodus und Wiederaufgreifen des medizinischen Implantats (10), wenn das medizinische Implantat aus dem Katheter (100) freigegeben wird, konfiguriert ist.

11. Medizinisches Implantat nach irgendeinem der vorhergehenden Ansprüche, wobei die Befestigungsvorrichtung (20) eine Verriegelungsverbindungsstruktur (50) zum Zusammenwirken mit einem entsprechenden Element des Katheters (100) bereitstellt.

## Revendications

1. Implant médical (10) destiné à une introduction dans un tissu humain ou animal, en particulier stimulateur cardiaque sans sonde, ayant une extrémité distale (14) et une extrémité proximale (12) opposée avec une fixation rigide (20) mise en place pour une interaction avec un élément de couplage (300) d'un cathéter (100) et faisant saillie depuis l'extrémité proximale (12) en direction d'un axe longitudinal de l'implant (10), la fixation (20) étant connectée de manière rigide à l'implant (10), **caractérisé en ce que**
la fixation (20) a généralement une forme en T, comprenant un montant (24) attaché à l'extrémité proximale (12) et aligné avec l'axe longitudinal de l'implant (10) et au moins une pièce en croix (30) disposée fixement au montant (24) de manière croisée par rapport à l'axe longitudinal, où une structure d'indentation (32) est agencée dans la pièce en croix (30).

2. Implant médical selon la revendication 1, dans lequel l'indentation (32) est agencée au centre de la pièce en croix (30) en direction de l'axe longitudinal du montant (24) et dans lequel l'indentation permet à l'élément d'attache (190) et/ou à un élément de recapture (190, 192), lorsqu'il est attaché à la fixation (20), de s'aligner avec l'axe longitudinal de l'implant médical (10).

3. Implant médical selon la revendication 1 ou 2, dans lequel la pièce en croix (30) est attachée au montant (24) de manière symétrique.

4. Implant médical selon la revendication 3, dans lequel la pièce en croix (30) a une configuration en deux lobes (34).

5. Implant médical selon l'une des revendications 1 à 3, dans lequel la pièce en croix (30) a une configuration en ballon de football ovale.

6. Implant médical selon l'une quelconque des revendications précédentes, dans lequel la pièce en croix (30) a une pointe arrondie au niveau de chacune de ses extrémités libres en direction radiale.

7. Implant médical selon la revendication 6, dans lequel la pièce en croix (30) a des pointes gonflées.

8. Implant médical selon l'une quelconque des revendications précédentes, dans lequel la pièce en croix (30) procure une surface plane au niveau de son extrémité libre (38) dans la direction longitudinale.

9. Implant médical selon la revendication 1, dans lequel la fixation (20), de préférence, l'extrémité libre (38), procure un élément fileté de fixation, de préférence un fil central (52) pour attacher un élément fileté correspondant du cathéter (100).

10. Implant médical selon l'une quelconque des revendications précédentes, dans lequel la fixation (20) est configurée pour un engagement primaire avec un élément de couplage (300) du cathéter (100) dans un mode d'engagement primaire de fonctionnement, ainsi que pour procurer une interface pour attacher un élément d'attache (190, 130) procuré par le cathéter (100) pour un mode de fonctionnement d'attache et pour la récupération de l'implant médical (10) lorsque l'implant médical est libéré du cathéter (100).

11. Implant médical selon l'une quelconque des revendications précédentes, dans lequel la fixation (20) procure une structure de connexion par un verrouillage par rotation (50) pour une interaction avec un élément correspondant du cathéter (100).
